# EUROPÄISCHE PATENTSCHRIFT

(11) **EP 3 095 384 B1**
(45) Veröffentlichungstag und Bekanntmachung des Hinweises auf die Patenterteilung: **01.01.2020**
(21) Anmeldenummer: 16000963.5
(22) Anmeldetag: 28.04.2016
(51) Int. Cl.: A61B 5/00, A61B 5/024, A61B 5/1455, A61B 5/145

(54) **VERFAHREN UND VORRICHTUNG ZUR NICHT-INVASIVEN BESTIMMUNG EINER MESSGRÖSSE EINES ANALYTEN IN EINEM BIOLOGISCHEN KÖRPER**
METHOD AND DEVICE FOR THE NON-INVASIVE DETERMINATION OF A MEASUREMENT PARAMETER OF AN ANALYTE IN A BIOLOGICAL BODY
PROCEDE ET DISPOSITIF DE DETERMINATION NON INVASIVE D'UNE GRANDEUR DE MESURE D'UN ANALYTE DANS UN CORPS BIOLOGIQUE

(30) Priorität: 19.05.2015 DE 102015006406
(43) Veröffentlichungstag der Anmeldung: 23.11.2016
(73) Patentinhaber: SAMTD GmbH & Co. KG, 90552 Röthenbach (DE)
(72) Erfinder: Tholl, Hans Dieter, 88682 Salem (DE); Glasmacher, Mathias, 68799 Reilingen (DE)
(74) Vertreter: Diehl Patentabteilung

(56) Entgegenhaltungen:
- EP-A1- 2 799 010
- EP-A2- 0 234 869
- WO-A1-01/50955
- WO-A1-2014/020611
- WO-A1-2014/116483
- US-A1- 2002 016 533
- US-B2- 8 277 384

## Beschreibung

Die Erfindung betrifft ein Verfahren zur nicht-invasiven Bestimmung der Konzentration eines Analyten in einem biologischen Körper, wobei automatisiert der Körper lokal mit Licht aus einem auf eine Absorptionssignatur des Analyten abgestimmten Wellenlängenbereich bestrahlt wird, und wobei wenigstens ein Teil des Lichts in den Körper eindringt und vom Analyten absorbiert wird. Die Erfindung betrifft weiter eine zur Durchführung des Verfahrens entsprechend ausgebildete Vorrichtung. Insbesondere beschäftigt sich die Erfindung mit einer nicht-invasiven Bestimmung des Blutzuckerspiegels.

Grundsätzlich ist es wünschenswert, biomedizinische Parameter in-vivo und nicht-invasiv bestimmen zu können. Damit können insbesondere für eine Diagnose erforderliche und wiederkehrend oder häufig zu überprüfende Parameter schmerzfrei und ohne einen Eingriff an einem menschlichen oder tierischen Körper ermittelt werden. Gerade eine nicht-invasive Bestimmung einer Messgröße eines im Körper enthaltenen Analyten gestaltet sich jedoch schwierig, da durch die Vielzahl von sich in ihren physikalischen und chemischen Parametern unterscheidenden Strukturen eines biologischen Körpers, wie Haut, Muskeln, Sehnen, Knochen, Gefäße, Fettgewebe und Organgewebe, der Erhalt von konkret einem Analyten zuordbaren Messsignalen erschwert ist. Zudem sind Messsignale aus dem Körperinneren grundsätzlich mit einem vergleichsweise schlechten Signal-zu-Rausch-Verhältnis belegt. Muss die Messgröße des Analyten für eine konkrete Struktur, beispielsweise in einem Blutgefäß ermittelt werden, so wird das Messergebnis zusätzlich durch die Umgebung verfälscht, wenn dort die Messgröße des Analyten einen anderen Wert aufweist. Interessierende Analyten sind beispielsweise Zucker, insbesondere Glucose, Alkohol, Drogen, Fette und Wasser, aber auch Hormone, Botenstoffe, Enzyme, Spurenelemente, Mineralien, Metalle, Medikamente und toxische Substanzen. Die Analyten können hierbei in fester, gasförmiger oder flüssiger Form vorliegen, wobei der Analyt insbesondere als eine Lösung in Körperflüssigkeiten oder in Körpergewebe gegeben sein kann.

Zu einer nicht-invasiven Ermittlung von biomedizinischen Parametern eignen sich bekanntermaßen insbesondere optische Methoden, die durch Streuung, Transmission, Absorption, Reflexion, Polarisation, Phasenänderung, Fluoreszenz, photoakustische Anregung oder photothermische Anregung in der Lage sind, die Anwesenheit des gesuchten Analyten zu detektieren. Je nach Methode kann aus dem Detektionssignal dann mit einer entsprechenden Messgenauigkeit ein Wert für die gewünschte Messgröße, wie beispielsweise ein Wert für eine Konzentration, bestimmt werden. Insbesondere kann nach einer spezifischen Absorption durch Einstrahlung eines auf eine Absorptionssignatur des Analyten abgestimmten Lichts bestimmter Wellenlänge ein für den Analyten selektives Messsignal erhalten werden, das eine quantitative Erfassung erlaubt.

Beispielsweise ist aus M.A. Pleitez et al., "In Vivo Noninvasive Monitoring of Glucose Concentration in Human Epidermis by Mid-Infrared Pulsed Photoacoustic Spectroscopy", Analytical Chemistry 2013, Bd. 85 (2), S. 1013 - 1020 eine photoakustische Methode zur Bestimmung der Konzentration von Glucose in menschlicher Epidermis bekannt. Dabei wird die Haut im Fingerprint-Bereich der Glucose mit Licht von Wellenlängen zwischen 8 µm und 10 µm, also im Bereich der Anregungsenergien von charakteristischen Ringdeformationsschwingungen, gepulst bestrahlt. Als Messsignal werden aus dem Körper austretende akustische Schwingungen detektiert, die als Folge der Absorption durch in interstitieller Flüssigkeit im Gewebe enthaltener Glucose entstehen. Licht in diesem Wellenlängenbereich dringt hierbei einige 10 µm in die Haut ein, so dass im interstitiellen Wasser die in der Epidermis enthaltene Glucose detektiert und bestimmt werden kann. Aus Z. Zalevsky, J. Garcia, "Laserbasierte biomedizinische Untersuchungen - simultan und kontaktlos", BioPhotonic 3, 2012, S. 30 - 33 ist weiter eine nicht-invasive Methode zur Bestimmung des Alkohol- und des Glucosespiegels in Blut bekannt, wobei durch Beobachtung von Speckle-Mustern Hautvibrationen vermessen werden. Dazu wird die Haut mit einem Laser beleuchtet und die durch Interferometrie gebildeten Speckle-Muster nachverfolgt.

Dazu wird weiter in N. Ozana et al., "Improved noncontact optical sensor for detection of glucose concentration and indication of dehydration level", Biomedical Optics Express 2014, Vol. 5, No. 6, S. 1926 - 1940 vorgeschlagen, die Sensitivität der Speckle-Interferometrie für Glucose dadurch zu erhöhen, dass zusätzlich zur Bestrahlung der Haut mit einem Laserstrahl ein Magnetfeld erzeugt wird, wobei die optische Aktivität des Glucose-Moleküls ausgenutzt wird. Über die Speckle-Muster werden blutpulsbedingte Vibrationen der Haut beobachtet. Aus einem maximalen Versatz des Speckle-Musters wird auf die Konzentration von Glucose in Blut geschlossen. Eine vergleichbare Methode ist in Y. Beiderman et al., "Demonstration of remote optical measurement configuration that correlates to glucose concentration in blood", Biomedical Optics Express, 2011, Vol. 2, No. 4, S. 858 - 870 beschrieben. Auch hier wird aus dem Versatz in auf den Puls- bzw. Herzschlag korrelierten Speckle-Mustern auf die Konzentration von Glucose in Blut geschlossen. Dies wird theoretisch dadurch erklärt, dass Glucose einen Einfluss auf die Viskosität des Blutes nimmt, wodurch die blutpulsbedingten Vibrationen der Haut eine Abhängigkeit von der Glucose-Konzentration zeigen. Diese Abhängigkeit kann über den Versatz der Speckle-Muster erfasst werden.

Ferner ist aus K.-U. Jagemann et al., "Application of Near-Infrared Spectroscopy for Non-Invasive Determination of Blood/Tissue Glucose Using Neural Networks", Zeitschrift für Physikalische Chemie, Bd. 191, 1995, S. 179 - 190 eine nicht-invasive Bestimmung von Glucose in Blut oder Gewebe mittels NIR-Spektroskopie bekannt. Dort wird mit Licht im nahen infraroten Spektralbereich eingestrahlt. Als Messsignal werden Spektren in diffuser Reflektion beobachtet. Zur Verbesserung des Messsignals ist weiter aus K. Yamakoshi et al. "Pulse Glucometry: A new Approach for Non-invasive Blood Glucose Measurement Using Instantaneous Differential Near Infrared Spectrophotometry", Journal of Biomedical Optics, Bd. 11 (5), 2006, S.1-11 bekannt, NIR-Spektren mit dem Herzschlag zu korrelieren. Aus Xinxin Guo et al., "Noninvasive glucose detection in human skin using wavelength modulated differential laser photothermal radiometry", Biomedical Optics Express, Bd. 3(11), 2012, S. 3012 - 3021 ist es ferner bekannt, die Glucose-Konzentration in Haut mittels eines photothermischen Up-Convertierungs-Prozesses durch gleichzeitige Einstrahlung von Laserlicht zweier diskreter Wellenlängen unter Beobachtung des differentiellen Emissionsspektrums zu ermitteln.

Die bisher bekannten Methoden zu einer in-vivo nicht-invasiven Bestimmung der Konzentration eines Analyten in einem biologischen Körper erreichen nicht die für klinische Anwendungen gewünschte Sensitivität und Spezifität. Einige dieser Methoden sind zudem aufgrund der Komplexität der erforderlichen Messapparaturen und aufgrund der für die Messung benötigten Zeit nicht geeignet, von Patienten zu einer eigenen regelmäßigen Kontrolle der entsprechenden Konzentration des Analyten herangezogen bzw. benutzt zu werden. Insbesondere gibt es zur Bestimmung der Blutzuckerkonzentration noch kein nicht-invasives Verfahren, welches von an Diabetes erkrankten Personen selbst zu einer regelmäßigen Kontrolle zuhause eingesetzt werden könnte. Gleichwohl wäre dies wünschenswert, da die bisherigen Verfahren regelmäßig eine gegebenenfalls schmerzhafte Blutentnahme erforderlich machen, bzw. eine in gewissen Situationen sinnhafte, in kurzen Zeitabständen wiederholte Messung nicht praktikabel ist.

Aus der US 2002/016533 A1, der WO 2014/116483 A1, der WO 01/50955 A1 und der EP 0 234 869 A2 ist jeweils bekannt, die Flussrate von Blut bzw. von roten Blutkörperchen mittels Analyse des Speckle-Kontrastes vom an den sich bewegenden Blutzellen reflektiertem Licht zu ermitteln.

Der Erfindung liegt daher die Aufgabe zugrunde, ein alternatives nicht-invasives Verfahren zur Bestimmung der Konzentration eines Analyten in einem biologischen Körper anzugeben, welches das Potential für eine Anwendung im Consumer-Markt oder im klinischen Alltag bietet, so dass der Patient eigenständig Messungen zur Kontrolle der jeweiligen Konzentration durchführen kann.

Weiter liegt der Erfindung die Aufgabe zugrunde, eine zur Durchführung des angegebenen Verfahrens geeignete Vorrichtung anzugeben, die die Möglichkeit zu einer Weiterentwicklung in ein Consumer-Produkt oder für den klinischen Alltag bietet.

Die erstgenannte Aufgabe wird erfindungsgemäß durch ein Verfahren zur nicht-invasiven Bestimmung der Konzentration eines Analyten in einem biologischen Körper gelöst, wobei automatisiert der Körper lokal mit Licht einer Lichtquelle aus einem auf eine Absorptionssignatur des Analyten abgestimmten Wellenlängenbereich zwischen 6 µm und 16 µm bestrahlt wird, wobei wenigstens ein Teil des Lichts in den Körper eindringt und vom Analyten absorbiert wird, wobei sich der Körper infolge der Absorption durch den Analyten zumindest lokal erwärmt und eine als Folge der Absorption auftretende Änderung in einem Speckle-Muster von am Körper gestreutem kohärentem Licht erfasst wird, und wobei aus der erfassten Änderung im Speckle-Muster auf einen Wert der Konzentration des Analyten geschlossen wird.

Die Erfindung geht dabei in einem ersten Schritt von der Überlegung aus, dass sich das Absorptionsspektrum eines insbesondere organischen Analyten in seinem Fingerprint-Bereich, der grob zwischen 6 µm und 16 µm liegt, charakteristisch von dem von Wasser unterscheidet. Allerdings sind die Absorptionskoeffizienten von Wasser auch in diesem Wellenlängenbereich hoch. Insofern dringt nur ein geringer Anteil des auf eine charakteristische Absorptionssignatur im Fingerprint-Bereich des Analyten abgestimmten Lichts in den biologischen Körper ein, um dort mit dem tieferliegenden Analyten wechselwirken zu können. Ein noch deutlich geringerer Anteil an Licht verlässt infolge der mit der Absorption verbundenen charakteristischen Rückstreuung den Körper und steht zur Detektion zur Verfügung. Das gewünschte Messsignal weist demnach grundsätzlich ein sehr niedriges Signal-zu-Rausch-Verhältnis auf.

In einem zweiten Schritt geht die Erfindung von der Erkenntnis aus, dass beobachtbare, für den Analyten an sich charakteristische Absorptionsspektrum bei Betrachtung in einer Streu-, Reflexions-, Emissions- oder Transmissionsgeometrie durch Wechselwirkung des Beobachtungslichts mit dem Körper an Aussagekraft verliert. Das Beobachtungslicht erfährt eine wellenlängenabhängige Dämpfung. Die Selektivität hinsichtlich des Analyten verringert sich.

In einem dritten Schritt gelangt die Erfindung zur Erkenntnis, dass das Problem des niedrigen Signal-zu-Rausch-Verhältnisses und der geringen Selektivität bei Beobachtung der an sich charakteristischen Absorption des Analyten umgangen werden kann, wenn ein Speckle-Muster beobachtet wird, welches in Relation zur charakteristischen Absorption steht. Dazu wird ausgenutzt, dass sich der biologische Körper infolge der spezifischen Absorption durch den Analyten zumindest lokal erwärmt, wodurch sich als Folge insbesondere eine zumindest lokale Deformation seiner Oberfläche, eine Veränderung der Streueigenschaften (Streukoeffizient, Winkelstreuung) in oberflächennahen Schichten und/oder eine Veränderung der Brechzahl in oberflächennahen Schichten zeigt/zeigen. Diese spezifisch mit dem Analyten und insbesondere mit seiner Konzentration im Zusammenhang stehenden Veränderungen an der Körperoberfläche oder in oberflächennahen Schichten des Körpers werden über eine beobachtete Änderung in dem Speckle-Muster erfasst. Aus dieser Änderung kann auf einen Wert der Konzentration des Analyten geschlossen werden. Änderungen im Speckle-Muster stehen damit in einem unmittelbaren Zusammenhang mit der Anwesenheit des Analyten, der über eine spezifische Absorption des eingestrahlten Lichts abgefragt wird.

Demgegenüber sehen die aus dem Stand der Technik bekannten Verfahren zu einer in-vivo-Bestimmung der Konzentration eines Analyten in einem biologischen Körper mittels Speckle-Interferometrie lediglich vor, eingestrahltes Licht ausschließlich zur Erzeugung des Speckle-Musters einzusetzen. Änderungen im Speckle-Muster werden an pulsierenden Hautoberflächen abgefragt. Eine Aussage über die zu bestimmende Konzentration des Analyten ergibt sich aus den beobachteten Änderungen im Speckle-Muster als Folge der Konzentration des Analyten im Blut, wodurch sich dessen Fließeigenschaften verändern. Da jedoch die Fließeigenschaften von Blut nicht ausschließlich von dem beobachteten Analyten abhängig sind, ist das vorliegend angegebene neuartige Verfahren wesentlich selektiver. Zugleich weist es auch eine höhere Sensitivität auf. Die Spezifität und Sensitivität des neuartigen Verfahrens ist im Übrigen auch gegenüber den eingangs beschriebenen optischen Verfahren verbessert, die Absorptionseigenschaften des Analyten abfragen.

Unter einem Speckle-Muster wird vorliegend im Übrigen das sich bei flächiger Bestrahlung einer Oberfläche mit kohärentem Licht im diffus gestreuten oder reflektierten Licht am Ort des Betrachters infolge unterschiedlicher Phasenlagen ergebende Muster mit charakteristischen Intensitätsmaxima und Intensitätsminima verstanden. Das durch Interferenz entstehende Muster ist äußerst sensitiv auf Veränderungen der Oberfläche oder in oberflächennahen Schichten.

Für den Wellenlängenbereich des eingestrahlten Lichts wird ein solcher Bereich ausgewählt, mit dem eine zur Bestimmung des spezifischen Analyten geeignete Eindringtiefe im Körper erreicht wird. Bevorzugt ist dieser Wellenlängenbereich im Hinblick auf das Absorptionsspektrum von Wasser für eine genügende Eindringtiefe günstig gewählt. Ein geeigneter Wellenlängenbereich ist insofern der sogenannte Fingerprint-Bereich von organischen Molekülen, also der Bereich der Energien charakteristischer Gerüststrukturschwingungen. Erfindungsgemäß wird daher der Körper mit Licht aus einem Wellenlängenbereich mit Wellenlängen zwischen 6 µm und 16 µm, bevorzugt im LWIR-Bereich (Long-Wavelength-Infrared) zwischen 8 µm und 15 µm, bestrahlt. Soll Glucose als Analyt bestimmt werden, so ist zweckmäßigerweise ein Bereich zwischen 7 µm und 11 µm gewählt, in dem das Glucose-Molekül charakteristische Ringdeformationsschwingungen zeigt. Durch eine insbesondere schmalbandige Wahl des eingestrahlten Lichts, die auf eine spezifische Absorptionssignatur des Analyten ausgerichtet ist, kann die Selektivität des Verfahrens weiter verbessert werden.

In einer weiter bevorzugten Ausgestaltung wird die Wellenlänge des eingestrahlten Lichts während der Messung variiert, wobei für verschiedene Wellenlängen jeweils die Änderung im Speckle-Muster erfasst wird. Insbesondere wird das für das eingestrahlte Licht vorgesehene Wellenlängenintervall von der niedrigsten zur höchsten Wellenlänge durchlaufen und für jede Wellenlänge die Änderung im Speckle-Muster bevorzugt mehrfach aufgenommen. Dadurch wird die Antwort des Messsystems mehrfach redundant ermittelt. Zusätzlich kann zweckmäßigerweise auch die Leistungsdichte des eingestrahlten Lichts während der Messung variiert und für verschiedene Leistungsdichten die Änderung im Speckle-Muster erfasst werden. Hierdurch wird die Redundanz bzw. Überbestimmtheit des Systems weiter vergrößert. Zur Durchführung der Messung werden insbesondere Bildsequenzen des Speckle-Musters bei verschiedenen Wellenlängen und/oder bei verschiedenen Leistungsdichten aufgenommen.

Bevorzugt wird die Änderung im Speckle-Muster abwechselnd bei einer Wellenlänge in einem Absorptionsmaximum und bei einer Wellenlänge zwischen den Absorptionsmaxima des Analyten erfasst. Durch eine Differenzauswertung der beobachteten Speckle-Muster kann auf die zu bestimmende Messgröße des Analyten geschlossen werden. Dadurch können Störeffekte minimiert werden bzw. sich aus dem Messergebnis herausrechnen lassen. Die Messung im Absorptionsminimum zeigt Artefakte, die nicht auf die Anwesenheit des Analyten zurückzuführen sind.

In einer vorteilhaften weiteren Ausgestaltung wird das Licht mit einer Modulationsfrequenz periodisch eingestrahlt. Auch hierdurch können Differenzialverfahren für die Auswertung der Messsignale genutzt werden. Die Auswertung geschieht dann durch eine Differenzbetrachtung des Speckle-Musters bei eingeschaltetem Anregungslicht und bei ausgeschaltetem Anregungslicht. Mit anderen Worten: es wird die spezifische Erwärmung der Körperoberfläche durch die Modulationsfrequenz zeitlich moduliert. Aus einer Bildsequenz des Speckle-Musters kann bevorzugt auch auf Relaxationszeiten der Körperoberfläche nach erfolgter Erwärmung geschlossen werden. Auch diese Abklingzeiten können zur Bestimmung der Messgröße des Analyten herangezogen werden. In einer alternativen Variante kann die spezifische Bestrahlung des Analyten beendet werden, und die zeitliche Entwicklung des Speckle-Musters analysiert werden. Dies gibt eine Aussage über die Entwicklung der Oberflächentemperatur mit der Zeit. Als Differenzbilder können hierbei in einer Bildsequenz aufeinander folgende Bilder des Speckle-Musters hinsichtlich einer Änderung ausgewertet werden.

In einer besonders geeigneten Messmethode wird die Erfassung der Änderung im Speckle-Muster auf die Modulationsfrequenz des periodisch eingestrahlten Lichts synchronisiert. Die Änderung im Speckle-Muster wird dabei vorteilhaft aus einer Differenzbetrachtung von Bildern des Speckle-Musters bei sich unterscheidenden Phasenlagen der Lichteinstrahlung gewonnen. Zweckmäßigerweise wird dabei über eine Mehrzahl von Bildern des Speckle-Musters gleicher Phasenlage gemittelt. Zusätzlich kann die Leistungsdichte des eingestrahlten Lichts wie vorbeschrieben zeitlich moduliert werden. Hierdurch kann das Signal-Rausch-Verhältnis des Messsignals weiter verbessert werden. Das dann gebildete Änderungssignal zeigt eine Modulation entsprechend der Modulationsfrequenz der Leistungsdichte. Auch die Informationen aus dieser Modulation können zur Bestimmung und Auswertung der Messgröße des Analyten herangezogen werden.

Da eine weitere periodische Eigenschaft in einem biologischen Körpers eines Tieres oder Menschen die Herzschlagfrequenz ist, wird bevorzugt die erfasste Änderung im Speckle-Muster zusätzlich oder alternativ korreliert zu einer Herzfrequenz des biologischen Körpers detektiert. Durch ein solches Verfahren wird Bezug genommen auf die pulsierende Eigenschaft des Systems als Folge des Herzschlags, was sich in variierenden Geometrien, Drücken und Temperaturen sowie in sich daraus ergebenden variierenden Konzentrationen des Analyten niederschlägt. Wird die Änderung im Speckle-Muster, beispielsweise bei modulierter Ein/Aus-Einstrahlung, bei Variation der optischen Leistungsdichte oder bei Variation der Wellenlänge, jeweils aus Bildern bei gleicher Phasenlage des Herzschlags erfasst, so werden derartige periodische Messunsicherheiten eliminiert.

In einer weiter bevorzugten Ausgestaltung wird eine Bildsequenz von Speckle-Mustern aufgenommen, wobei die Änderung im Speckle-Muster durch einen Vergleich von Bildern aus der aufgenommenen Bildsequenz erfasst wird. Die zur Erfassung der Änderung im Speckle-Muster herangezogenen Bilder werden entsprechend des jeweils verwendeten Modulationsverfahrens des eingestrahlten Lichts aus der aufgenommenen Bildsequenz ausgewählt. Insbesondere werden Hell-/Dunkel-Differenzbilder ausgewählt, die sich auf die Leistungsdichte, die Wellenlänge oder auf ein- bzw. ausgeschaltetes Licht zur Anregung des Analyten beziehen. In solchen Differenzbildern ist die durch den Analyten bedingte größtmögliche Änderung im Speckle-Muster zu erwarten, so dass eine solche Auswertung eine hohe Spezifität und Sensitivität für den zu untersuchenden Analyten hat. Zugleich werden durch die Bildung von Differenzbildern Beleuchtungsartefakte und Offset-Effekte verringert.

Bevorzugt wird die Änderung im Speckle-Muster als ein Kontrastwert in einem Differenzbild ermittelt. Im Differenzbild wird dazu beispielsweise innerhalb eines ausgewählten Bildausschnitts eine Analyse hinsichtlich der Standardabweichung und hinsichtlich des Mittelwerts der betrachteten Pixel-Werte durchgeführt. Zur Betrachtung des Speckle-Musters wird hierbei zweckmäßigerweise eine Bildaufnahmeeinrichtung, wie beispielsweise ein CCD-Detektor oder eine digitale Bildaufnahmekamera, verwendet. Zur Auswertung kann im Differenzbild sowohl ein globaler als auch ein lokaler Kontrast herangezogen werden. Der globale Kontrast kann beispielsweise als größter Grauwertunterschied im aufgenommenen Bild ermittelt werden. Ein lokaler Kontrast kann als durchschnittlicher Grauwertunterschied zwischen benachbarten Pixeln in einem Bildbereich vorgegebener Größe berechnet werden.

Zweckmäßigerweise werden die Bilder der Bildsequenz mit einer vorgegebenen Bildwiederholrate aufgenommen, wobei die Bildwiederholrate zur Modulationsfrequenz des periodisch eingestrahlten Lichts und/oder zur Herzfrequenz des biologischen Körpers synchronisiert wird. Auf diese Weise weisen die Bilder der Bildsequenz eine feste Phasenbeziehung zur Modulation des periodisch eingestrahlten Lichts und/oder zur Herzfrequenz auf. Über diese Phasenbeziehung wird es möglich, Bilder der Bildsequenz selektiv auszuwählen und zur Differenzauswertung heranzuziehen, wodurch das Signal-zu-Rausch-Verhältnis des Messsignals verbessert wird.

In einer weiter bevorzugten Ausgestaltung wird die Änderung im Speckle-Muster aus der aufgenommenen Bildsequenz als eine Funktion der Zeit erfasst. Hierdurch können wie bereits ausgeführt Aussagen über Relaxationszeiten des Körpers bzw. der Körperoberfläche gewonnen werden. Insgesamt wird dadurch das beobachtete System überbestimmt erfasst, wodurch die Spezifität und Selektivität hinsichtlich des untersuchten Analyten weiter verbessert wird.

Der Kontrast im Differenzbild des Speckle-Musters kann zur Bestimmung der Messgröße des Analyten weiter als Funktion der eingestrahlten Wellenlänge und/oder als Funktion des Modulationsindex bei zusätzlicher Variation der Leistungsdichte ermittelt und entsprechend ausgewertet werden.

Um das Signal-zu-Rausch-Verhältnis weiter zu verbessern, wird bevorzugt der Körper mit polarisiertem Licht bestrahlt. Hierdurch kann der direkte Fresnel-Reflex beispielsweise durch Einsatz eines entsprechenden Polarisationsfilters diskriminiert werden. Dies ist insbesondere dann von Vorteil, wenn das selektiv eingestrahlte Licht sowohl zur Anregung des Analyten als auch zur Erzeugung des Speckle-Musters herangezogen wird. In diesem Fall ist neben der Anregungslichtquelle keine weitere Messlichtquelle notwendig. Auch die aus tieferen Körperschichten reemittierte, depolarisierte Strahlung würde Speckle-Muster erzeugen. Bevorzugt wird bei Einstrahlung von polarisiertem Licht ein Polarisationsdiskriminator eingesetzt bzw. eine Polarisationsanalyse des beobachteten Lichtes bzw. Speckle-Musters durchgeführt; So kann das Speckle-Muster abhängig von der Polarisation des am Körper gestreuten Lichts beobachtet und die Änderung im Speckle-Muster jeweils für eine bestimmte Polarisation erfasst werden.

Vorteilhafterweise wird am Ort der Bestrahlung des Körpers mit Licht zusätzlich ein Magnetfeld erzeugt. Ein solches Magnetfeld ist von Vorteil, wenn der untersuchte Analyt einen vergleichsweise großen Faraday-Effekt zeigt. In Anwesenheit eines äußeren Magnetfeldes wird dann durch den untersuchten Analyt eine spezifische Drehung der Polarisationsrichtung des eingestrahlten Lichts bewirkt. Im Zusammenhang mit einem Polarisationsanalysator oder Polarisationsdiskriminator kann dann die Spezifität des Messverfahrens weiter vergrößert werden. Insbesondere zeigt Glucose als Analyt eine vergleichsweise große Verdet-Konstante, die maßgeblich für den Drehwinkel der Polarisationsänderung ist.

Bevorzugt wird der Körper mit einer durchstimmbaren Lichtquelle, insbesondere mit einem schmalbandigen Halbleiterlaser bestrahlt. Insbesondere ein Quantenkaskadenlaser ist in der Lage, mit hoher Güte innerhalb des Fingerprint-Bereichs schmalbandig eine Wellenlänge zu emittieren. Zugleich weist ein derartiger Laser eine Durchstimmbarkeit von etwa 20% der Zentralwellenlänge auf. Beispielsweise kann ein Quantenkaskadenlaser eingesetzt sein, der durchstimmbar Licht mit einer Wellenlänge zwischen 7 µm und 11 µm zu emittieren. In diesem Bereich liegen charakteristische Absorptionsbanden von Glucose als einem bevorzugt zu beobachtenden Analyten.

In einer anderen Variante wird zur Beleuchtung des Körpers eine breitbandig emittierende Lichtquelle eingesetzt. Der jeweils einzustrahlende Wellenlängenbereich kann dann beispielsweise durch Filterung selektiv ausgewählt werden. Als eine im IR-Bereich oder in einem IR-Teilbereich emittierende Lichtquelle kann insbesondere eine Wärmequelle eingesetzt sein. Eine solche Wärmequelle erlaubt eine ad hoc Einstrahlung eines ganzen Wellenlängenbereichs. Allerdings sind die Intensitäten breitbandig emittierender Lichtquellen in der Regel gegenüber durchstimmbaren, bei einer konkreten Wellenlänge emittierenden Lichtquelle niedriger und zeigen eine geringere Güte. Zudem muss die Wärmestrahlung der emittierenden Lichtquelle gut separiert werden.

Wie beschrieben kann das Messverfahren zur Bestimmung der Messgröße eines Analyten mit einer einzigen Lichtquelle durchgeführt werden, wenn diese sowohl in einem Absorptionsbereich des Analyten emittiert als auch zur Erzeugung eines Speckle-Musters geeignet ist. Dies ist dann der Fall, wenn zur Erzeugung des auf die Absorptionssignatur des Analyten abgestimmten Lichts eine erste kohärente Lichtquelle verwendet wird, und wenn das Speckle-Muster in dem vom Körper rückgestreuten Licht der ersten Lichtquelle beobachtet wird. Ein geeigneter Halbleiterlaser erfüllt diese Bedingung, so dass dasselbe Licht sowohl zur Anregung des Analyten als auch zur Erzeugung des Speckle-Musters herangezogen werden kann.

In einer weiteren vorteilhaften Ausgestaltung wird der Körper mit Licht einer zweiten kohärenten Lichtquelle, insbesondere im sichtbaren Spektralbereich, bestrahlt, wobei das Speckle-Muster in dem vom Körper rückgestreuten Licht der zweiten Lichtquelle beobachtet wird. In diesem Fall wird eine zweite Lichtquelle zur Erzeugung des Speckle-Musters verwendet. Über das Speckle-Muster werden dann ausschließlich lokale Deformationen auf der Oberfläche des Körpers beobachtet. Das Licht der ersten Lichtquelle kann aus dem beobachteten Strahlengang leicht mittels eines Filters ausgeblendet werden.

Vorteilhafterweise wird als Analyt Glucose betrachtet und als Messgröße der Glucose deren Konzentration bestimmt. Insbesondere kann das Verfahren insofern angewendet werden, um den Blutzuckerspiegel, also die Konzentration von Glucose im Blut, zu ermitteln. Zur jeweiligen Messung am Körper eignet sich insbesondere das Handgelenk, ein Unterarm oder ein Unterschenkel, da dort oberflächennahe Blutgefäße verlaufen. Durch Einstrahlung von Licht im Fingerprint-Bereich gelangt man über verschiedene Hautschichten ohne Glucose insbesondere in Gefäße (mit Glucose). Über die Änderung im Speckle-Muster der Hautoberfläche kann auf den jeweiligen Glucose-Gehalt in Blut zurückgeschlossen werden.

Bevorzugt umfasst die Ermittlung des Wertes der Messgröße in einer bestimmten Struktur des Körpers eine innere Kalibrierung durch Berücksichtigung wenigstens eines weiteren Wertes der Messgröße an einem anderen Beobachtungsort des Körpers. Bei einem Analyten in Blut bieten hierzu gegebenenfalls die verschiedenen Konzentrationen in Arterien und Venen eine geeignete Möglichkeit zu einer Kalibrierung. Auch kann die Tatsache ausgenutzt werden, dass die Konzentrationen des Analyten im interstitiellen Wasser und in Blut zueinander korreliert sind. Auch kann der Patient zu einer inneren Kalibrierung insbesondere im Falle von Glucose den Analyten zu sich nehmen und nachfolgend der zeitliche Verlauf des Anstiegs der Konzentration des Analyten im interstitiellen Wasser und in Blut beobachtet werden.

Die zweitgenannte Aufgabe wird erfindungsgemäß gelöst durch eine Vorrichtung zur nicht-invasiven Bestimmung einer Messgröße eines Analyten in einem biologischen Körper, umfassend wenigstens eine erste Lichtquelle zur Bestrahlung des Körpers mit Licht aus einem auf eine Absorptionssignatur des Analyten abgestimmten Wellenlängenbereich, eine Speckle-Einrichtung zur Erzeugung und Beobachtung eines Speckle-Musters von am Körper gestreutem kohärentem Licht, und eine Steuereinheit, die eingerichtet ist, eine Änderung in dem beobachteten Speckle-Muster zu erfassen und aus der erfassten Änderung auf einen Wert der Messgröße des Analyten zu schließen.

Weitere vorteilhafte Ausgestaltungen ergeben sich aus den folgenden Varianten und aus den auf die Vorrichtung gerichteten Unteransprüchen. Dabei können die für das Verfahren jeweils genannten Vorteile sinngemäß auf die Vorrichtung übertragen werden.

Bevorzugt ist die erste Lichtquelle in einem IR-Teilbereich, insbesondere in einem Wellenlängenbereich mit Wellenlängen zwischen 6 und 16 µm, durchstimmbar ist.

Weiter bevorzugt ist die erste Lichtquelle eingerichtet, Licht mit variabler Wellenlänge zu emittieren, und die Steuereinheit ist eingerichtet, die erste Lichtquelle zu einer Variation der Wellenlänge des eingestrahlten Lichts während der Messung anzusteuern und für verschiedene Wellenlängen jeweils die Änderung im Speckle-Muster zu erfassen.

Zweckmäßigerweise ist die erste Lichtquelle eingerichtet, Licht mit variabler Leistungsdichte zu emittieren, und die Steuereinheit ist eingerichtet, die Lichtquelle zu einer Variation der Leistungsdichte des eingestrahlten Lichts während der Messung zu variieren und für verschiedene Leistungsdichten jeweils die Änderung im Speckle-Muster zu erfassen.

Vorteilhaft ist die Lichtquelle periodisch mit einer Modulationsfrequenz betreibbar. Hierfür von Vorteil ist es weiter, wenn die Steuereinheit eingerichtet ist, die Änderung im Speckle-Muster synchron zur Modulation zu erfassen.

Bevorzugt ist ein Herzfrequenzsensor umfasst, wobei die Steuereinheit eingerichtet ist, die Änderung im Speckle-Muster synchron zum Herzschlag des biologischen Körpers zu erfassen.

In einer weiter bevorzugten Alternative ist die Speckle-Einrichtung eingerichtet ist, eine Bildsequenz von Speckle-Bildern zu erfassen, und die Steuereinheit ist eingerichtet, die Änderung im Speckle-Muster durch einen Vergleich von Bildern aus der aufgenommenen Bildsequenz zu erfassen.

Vorzugsweise ist die Steuereinheit eingerichtet, die Änderung im Speckle-Muster als einen Kontrastwert in einem Differenzbild zu ermitteln.

Zweckmäßigerweise ist die Speckle-Einrichtung eingerichtet, die Bildsequenz von Speckle-Bildern mit einer Bildwiederholrate zu erfassen, wobei die Steuereinheit eingerichtet ist, die Bildwiederholrate zur Modulationsfrequenz und/oder zur Herzfrequenz des biologischen Körpers zu synchronisieren.

Die Speckle-Einrichtung ist weiter vorteilhaft eingerichtet, die Änderung im Speckle-Muster aus der aufgenommenen Bildsequenz als eine Funktion der Zeit zu erfassen.

In einer vorteilhaften Ausführungsvariante ist die Steuereinheit eingerichtet, als Messgröße die Konzentration von Glucose zu bestimmen.

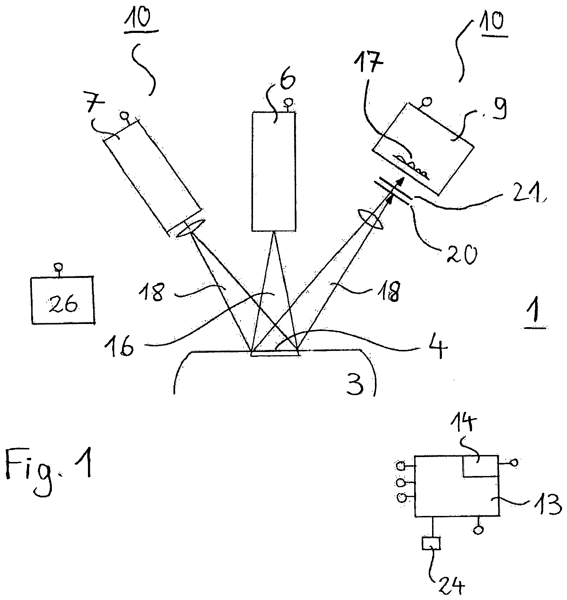

Ein Ausführungsbeispiel der Erfindung wird anhand einer Zeichnung näher erläutert. Dabei zeigt die einzige Figur 1 schematisch eine Vorrichtung 1 zur nicht-invasiven Bestimmung einer Messgröße eines Analyten in einem menschlichen Körper mittels des vorbeschriebenen Verfahrens. Insbesondere ist die dargestellte Vorrichtung 1 zur Bestimmung der Konzentration von Glucose in Blut ausgebildet. Als Teil des menschlichen Körpers 3 wird beispielsweise ein Unterarm mit auf eine spezifische Absorptionssignatur des Analyten, hier Glucose, abgestimmten Licht bestrahlt. Insbesondere ist hierbei die Hautoberfläche 4 von Interesse.

Die Vorrichtung 1 umfasst als eine erste Lichtquelle 6 zur Bestrahlung des Körpers 3 mit Licht 16 im IR-Spektralbereich einen durchstimmbaren Halbleiterlaser, insbesondere einen Quantenkaskadenlaser. Der Halbleiterlaser emittiert durchstimmbar Licht mit Wellenlängen zwischen 8 µm und 11 µm, also im Fingerprint-Bereich des Glucose-Moleküls. In diesem Bereich werden charakteristische Ringdeformationsschwingungen angeregt.

Weiter umfasst die Vorrichtung als eine zweite Lichtquelle 7 einen Speckle-Laser, der die Hautoberfläche 4 des Unterarms 3 kontinuierlich mit Licht 18 einer festen Wellenlänge im sichtbaren Spektralbereich beleuchtet. Das von der Hautoberfläche 4 gestreute Licht 18 der zweiten Lichtquelle 7 wird von einer Kamera 9 beobachtet. Aufgrund unterschiedlicher Phasenlagen der am Körper 3 gestreuten Lichtstrahlen resultiert am Ort der Kamera 9 ein Speckle-Muster 17, welches aufgrund Interferenz charakteristische Intensitätsminima und Intensitätsmaxima aufweist. Das Speckle-Muster 17 ist hochsensitiv auf Veränderungen der Hautoberfläche 4, wie z.B. auf durch Einstrahlung des Anregungslichts bedingte Deformationen. Die zweite Lichtquelle 7, die Kamera 9 und die entsprechenden Abbildungsoptiken im Strahlengang des Lichts 18 stellen gemeinsam eine Speckle-Einrichtung 10 dar, über die ein Speckle-Muster 17 von an der Hautoberfläche 4 reflektiertem Licht 18 erzeugt und beobachtet werden kann.

Durch Einstrahlung des Lichts 16 wird im Körper 3 und insbesondere in hautnahen Blutgefäßen enthaltene Glucose im Fingerprint-Bereich spezifisch angeregt. Infolgedessen kommt es im Bereich der Einstrahlung zu einer lokalen Erwärmung im Körper 3, was zu einer charakteristischen Deformation der Hautoberfläche 4 führt. Diese charakteristische Deformation 4 wird als eine Änderung im Speckle-Muster 17 in den von der Kamera 9 aufgenommenen Bildsequenzen erkannt.

Zur Erfassung der Änderung im Speckle-Muster 17 ist eine Steuereinheit 13 vorgesehen. Diese ist über entsprechende Steuer- und Datenleitungen mit den Lichtquellen 6, 7 bzw. der Kamera 9 verbunden. Die Steuereinheit 13 steuert die erste Lichtquelle 6 zu einer periodischen Bestrahlung des Körpers 3 an. Über die periodische Bestrahlung mit einer vorgegebenen Modulationsfrequenz wird die Absorption durch Glucose und damit als Folge die Deformation der Hautoberfläche 4 zeitlich moduliert. Mit der Modulationsfrequenz wird die Bildwiederholrate der Kamera 9 synchronisiert. Die Änderung im Speckle-Muster 17 wird durch Differenzbildung von Bildern der Bildsequenz ermittelt, die bei eingeschalteter Lichtquelle 6 und bei ausgeschalteter Lichtquelle 6 aufgenommen sind. Zusätzlich wird die Leistungsdichte der Lichtquelle 6 moduliert. Weiter wird der von der Lichtquelle 6 erzeugbare Wellenlängenbereich durchlaufen. Die Änderung im Speckle-Muster 17 wird als Kontrast im Differenzbild erfasst. Der Kontrast im Differenzbild wird Maß für die Konzentration der Glucose herangezogen.

Zusätzlich kann die Modulation im erfassten Kontrast als Folge der modulierten optischen Leistung erfasst und ausgewertet. Ebenso kann die Änderung im Differenzbild wellenlängenspezifisch ausgewertet werden. Durch diese zusätzlichen Informationen kann die Auswertegenauigkeit weiter erhöht werden.

Die zweite Lichtquelle 7 erzeugt polarisiertes Licht 18. Über einen Spektralfilter 20 wird Störlicht ausgeblendet. Mittels des Polarisationsdiskriminators 21 erfolgt eine Polarisationsanalyse des rückgestreuten Lichts 18, so dass auch eine polarisationsabhängige Erfassung des Speckle-Musters 17 ermöglicht ist.

Weiter umfasst die Vorrichtung 1 einen Herzfrequenzsensor 24. Die Steuereinheit 13 ist insbesondere dazu ausgebildet, die Bildwiederholrate der Kamera 9 auf die erfasste Herzfrequenz zu synchronisieren. Durch Beobachtung von Bildern bei jeweils fester Phasenlage zur Herzfrequenz können störende Messfehler durch die pulsierende Hautoberfläche 4 eliminiert werden. Zusätzlich ist ein Magnetfeldgenerator 26 umfasst. Wird über den Magnetfeldgenerator 26 ein Magnetfeld in den Körper 3 eingestreut, so führt Glucose aufgrund des relativ hohen Verdet-Faktors zu einer spezifischen Drehung der Polarisationsrichtung des eingestrahlten Lichts 18. Durch eine Polarisationsanalyse kann das Speckle-Muster 17 dann zusätzlich selektiv aus tieferen Hautschichten beobachtet und analysiert werden.

### Bezugszeichenliste

- 1: Vorrichtung
- 3: biologischer Körper
- 4: Oberfläche
- 6: erste Lichtquelle
- 7: zweite Lichtquelle
- 9: Kamera
- 10: Speckle-Einrichtung
- 13: Steuereinheit
- 14: Ausgabeeinheit
- 16: Licht
- 17: Speckle-Muster
- 18: Licht
- 20: Spektralfilter
- 21: Polarisationsdiskriminator
- 24: Herzfreqzenzsensor
- 26: Magnetfeldgenerator

## Patentansprüche

1. Verfahren zur nicht-invasiven Bestimmung der Konzentration eines organischen Analyten in einem biologischen Körper (3), wobei automatisiert der Körper (3) lokal mit Licht (16) aus einem auf eine Absorptionssignatur des Analyten, nämlich dessen Fingerprint-Bereich, abgestimmten Wellenlängenbereich zwischen 6 µm und 16 µm bestrahlt wird, wobei wenigstens ein Teil des Lichts in den Körper (3) eindringt und vom Analyten absorbiert wird, wobei sich der Körper (3) infolge der Absorption durch den Analyten zumindest lokal erwärmt und eine als Folge auftretende Änderung in einem Speckle-Muster (17) von am Körper (3) gestreutem kohärentem Licht (16, 18) erfasst wird, und wobei aus der erfassten Änderung im Speckle-Muster (17) auf einen Wert der Konzentration des Analyten geschlossen wird.

2. Verfahren nach Anspruch 1,
wobei als Folge der Absorption durch den Analyten eine Deformation der Oberfläche (4) des Körpers (3), eine Veränderung der Streueigenschaften oberflächennaher Bereiche des Körpers (3) und/oder eine Veränderung der Brechzahl oberflächennaher Bereiche des Körpers (3) auftritt/auftreten, und als Änderung im Speckle-Muster (17) erfasst wird.

3. Verfahren nach Anspruch 1 oder 2,
wobei der Körper (3) mit Licht (16) aus einem Wellenlängenbereich mit Wellenlängen zwischen 7 µm und 11 µm bestrahlt wird.

4. Verfahren nach einem der vorhergehenden Ansprüche,
wobei die Wellenlänge des eingestrahlten Lichts (16) während der Messung variiert wird und für verschiedene Wellenlängen jeweils die Änderung im Speckle-Muster (17) erfasst wird,
wobei bevorzugt vorgesehen ist, dass das Licht (16) abwechselnd mit einer Wellenlänge in einem Absorptionsmaximum des Analyten und mit einer Wellenlänge zwischen den Absorptionsmaxima des Analyten eingestrahlt wird.

5. Verfahren nach einem der vorhergehenden Ansprüche,
wobei die Leistungsdichte des eingestrahlten Lichts (16) während der Messung variiert wird, und wobei für verschiedene Leistungsdichten jeweils die Änderung im Speckle-Muster (17) erfasst wird.

6. Verfahren nach einem der vorhergehenden Ansprüche,
wobei das Licht (16) mit einer Modulationsfrequenz periodisch eingestrahlt wird und insbesondere vorgesehen ist, dass die Erfassung der Änderung im Speckle-Muster (17) auf die Modulationsfrequenz des periodisch eingestrahlten Lichts (16) synchronisiert wird.

7. Verfahren nach einem der vorhergehenden Ansprüche,
wobei die Änderung im Speckle-Muster (17) synchron zu einer Herzfrequenz des biologischen Körpers (3) erfasst wird.

8. Verfahren nach einem der vorhergehenden Ansprüche,
wobei eine Bildsequenz von Speckle-Mustern (17) aufgenommen wird und die Änderung im Speckle-Muster (17) durch einen Vergleich von Bildern aus der aufgenommenen Bildsequenz erfasst wird,
wobei die Änderung im Speckle-Muster (17) bevorzugt als ein Kontrastwert in einem Differenzbild ermittelt wird,
und wobei insbesondere vorgesehen ist, dass die Bilder der Bildsequenz mit einer Bildwiederholrate aufgenommenen werden und die Bildwiederholrate zur Modulationsfrequenz des periodisch eingestrahlten Lichts (16) und/oder zur Herzfrequenz des biologischen Körpers (3) synchronisiert wird,
und wobei die Änderung im Speckle-Muster (17) aus der aufgenommenen Bildsequenz bevorzugt als eine Funktion der Zeit erfasst wird.

9. Verfahren nach einem der vorhergehenden Ansprüche,
wobei der Körper (3) mit polarisiertem Licht (16) bestrahlt wird.

10. Verfahren nach einem der vorhergehenden Ansprüche,
wobei das Speckle-Muster (17) abhängig von der Polarisation des am Körper (3) gestreuten Lichts (16, 18) beobachtet wird, und wobei die Änderung im Speckle-Muster (17) jeweils für eine bestimmte Polarisation erfasst wird.

11. Verfahren nach einem der vorhergehenden Ansprüche,
wobei am Ort der Bestrahlung des Körpers (3) mit Licht (16) zusätzlich ein Magnetfeld erzeugt wird.

12. Verfahren nach einem der vorhergehenden Ansprüche,
wobei zur Erzeugung des auf die Absorptionssignatur des Analyten abgestimmten Lichts (16) eine durchstimmbare Lichtquelle (6), insbesondere ein Halbleiterlaser, verwendet wird.

13. Verfahren nach einem der vorhergehenden Ansprüche,
wobei zur Erzeugung des auf die Absorptionssignatur des Analyten abgestimmten Lichts (16) eine erste kohärente Lichtquelle (6) verwendet wird, und wobei das Speckle-Muster (17) in dem vom Körper (3) rückgestreuten Licht (16) der ersten Lichtquelle (6) beobachtet wird.

14. Verfahren nach einem der vorhergehenden Ansprüche,
wobei der Körper (3) mit Licht (18) einer zweiten kohärenten Lichtquelle (7), insbesondere im sichtbaren Spektralbereich, bestrahlt wird, und wobei das Speckle-Muster (17) in dem vom Körper (3) rückgestreuten Licht (18) der zweiten Lichtquelle (7) beobachtet wird.

15. Verfahren nach einem der vorhergehenden Ansprüche,
wobei als Analyt Glucose betrachtet wird.

16. Vorrichtung (1) zur nicht-invasiven Bestimmung der Konzentration eines organischen Analyten in einem biologischen Körper (3), umfassend wenigstens eine erste Lichtquelle (6) zur Bestrahlung des Körpers (3) mit Licht (16) aus einem auf eine Absorptionssignatur des Analyten, nämlich dessen Fingerprint-Bereich, abgestimmten Wellenlängenbereich zwischen 6 µm und 16 µm, eine Speckle-Einrichtung (10) zur Erzeugung und Beobachtung eines Speckle-Musters (17) von am Körper (3) gestreutem kohärentem Licht (16, 18), und eine Steuereinheit (13), die eingerichtet ist, eine Änderung in dem beobachteten Speckle-Muster (17) zu erfassen und aus der erfassten Änderung auf einen Wert der Konzentration des Analyten zu schließen,
wobei die Steuereinheit (13) zur Durchführung eines Verfahrens mit den Merkmalen nach einem der Ansprüche 1 bis 15 eingerichtet und ausgebildet ist.

17. Vorrichtung (1) nach Anspruch 16,
wobei die erste Lichtquelle (6) zur Emission von polarisiertem Licht (16) eingerichtet ist.

18. Vorrichtung (1) nach Anspruch 16 oder 17,
wobei die Speckle-Einrichtung (10) einen Polarisationsdiskriminator (21) umfasst, und wobei die Steuereinheit (13) eingerichtet ist, die Änderung im Speckle-Muster (17) jeweils für eine bestimmte Polarisation zu erfassen.

19. Vorrichtung (1) nach einem der Ansprüche 16 bis 18,
wobei zusätzlich ein Magnetfeldgenerator (26) zur Erzeugung eines Magnetfelds am Ort der Bestrahlung des Körpers (3) mit Licht (16) umfasst ist.

20. Vorrichtung (1) nach einem der Ansprüche 16 bis 19,
wobei als erste Lichtquelle (6) eine durchstimmbare Lichtquelle (6), insbesondere ein Halbleiterlaser, umfasst ist.

21. Vorrichtung (1) nach einem der Ansprüche 16 bis 20,
wobei die erste Lichtquelle (6) zur Erzeugung kohärenten Lichts (16) ausgebildet ist, und wobei die Speckle-Einrichtung (10) eingerichtet ist, das Speckle-Muster (17) in dem am Körper (3) gestreuten Licht (16) der ersten Lichtquelle (6) zu erzeugen und zu beobachten.

22. Vorrichtung (1) nach einem der Ansprüche 16 bis 21,
wobei eine zweite Lichtquelle (7) zur Erzeugung kohärenten Lichts (18), insbesondere im sichtbaren Spektralbereich, umfasst ist, und wobei die Speckle-Einrichtung (10) eingerichtet ist, das Speckle-Muster (17) in dem am Körper (3) gestreuten Licht (18) der zweiten Lichtquelle (7) zu erzeugen und zu beobachten.

## Claims

1. Method for non-invasive determination of the concentration of an organic analyte in a biological body (3), wherein, in an automated manner, the body (3) is irradiated locally by light (16) from a wavelength range of between 6µm and 16µm matched to an absorption signature of the analyte, namely the fingerprint range thereof,
wherein at least some of the light penetrates into the body (3) and is absorbed by the analyte,
wherein the body (3) heats up at least locally as a result of the absorption by the analyte and a consequently occurring change in a speckle pattern (17) of coherent light (16, 18) scattered at the body (3) is detected, and wherein a value of the concentration of the analyte is deduced from the detected change in the speckle pattern (17).

2. Method according to Claim 1,
wherein a deformation of the surface (4) of the body (3), a change in the scattering properties of regions of the body (3) close to the surface and/or a change in the refractive index of regions of the body (3) close to the surface occurs/occur as a consequence of the absorption by the analyte and this is detected as a change in the speckle pattern (17).

3. Method according to Claim 1 or 2,
wherein the body (3) is irradiated by light (16) from a wavelength range with wavelengths between 7 µm and 11 µm.

4. Method according to one of the preceding claims,
wherein the wavelength of the radiated-in light (16) is varied during the measurement and the change in the speckle pattern (17) is detected for different wavelengths in each case,
wherein it is preferably provided that the light (16) is alternately radiated-in with a wavelength in an absorption maximum of the analyte and with a wavelength between the absorption maxima of the analyte.

5. Method according to one of the preceding claims,
wherein the power density of the radiated-in light (16) is varied during the measurement and wherein the change in the speckle pattern (17) is detected for different power densities in each case.

6. Method according to one of the preceding claims,
wherein the light (16) is radiated-in periodically with a modulation frequency and it is provided in particular that the detection of the change in the speckle pattern (17) is synchronized with the modulation frequency of the light (16) radiated-in periodically.

7. Method according to one of the preceding claims,
wherein the change in the speckle pattern (17) is detected synchronously with a heart rate of the biological body (3).

8. Method according to one of the preceding claims,
wherein an image sequence of speckle patterns (17) is recorded and the change in the speckle pattern (17) is detected by a comparison of images from the recorded image sequence,
wherein the change in the speckle pattern (17) is preferably established as a contrast value in a difference image,
and wherein it is provided in particular that the images in the image sequence are recorded with an image refresh rate and the image refresh rate is synchronized with the modulation frequency of the light (16) radiated-in periodically and/or with the heart rate of the biological body (3),
and wherein the change in the speckle pattern (17) from the recorded image sequence is preferably detected as a function of time.

9. Method according to one of the preceding claims,
wherein the body (3) is irradiated by polarized light (16).

10. Method according to one of the preceding claims,
wherein the speckle pattern (17) is observed in a manner dependent on the polarization of the light (16, 18) scattered on the body (3) and wherein the change in the speckle pattern (17) is determined for a specific polarization in each case.

11. Method according to one of the preceding claims,
wherein a magnetic field is additionally generated at the location where the body (3) is irradiated by light (16).

12. Method according to one of the preceding claims,
wherein a tunable light source (6), in particular a semiconductor laser, is used to generate the light (16) matched to the absorption signature of the analyte.

13. Method according to one of the preceding claims,
wherein a first coherent light source (6) is used to generate the light (16) matched to the absorption signature of the analyte and wherein the speckle pattern (17) is observed in the light (16) of the first light source (6) scattered back from the body (3).

14. Method according to one of the preceding claims,
wherein the body (3) is irradiated by light (18) from a second coherent light source (7), in particular in the visible spectral range, and wherein the speckle pattern (17) is observed in the light (18) of the second light source (7) scattered back from the body (3).

15. Method according to one of the preceding claims,
wherein glucose is considered as analyte.

16. Apparatus (1) for non-invasive determination of the concentration of an organic analyte in a biological body (3), comprising at least a first light source (6) for irradiating the body (3) with light (16) from a wavelength range of between 6µm and 16µm matched to an absorption signature of the analyte, namely the fingerprint range thereof, a speckle device (10) for generating and observing a speckle pattern (17) of coherent light (16, 18) scattered at the body (3) and a control unit (13) configured to detect a change in the observed speckle pattern (17) and deduce a value of the concentration of the analyte from the detected change,
wherein the control unit (13) is configured and embodied to carry out a method with the features according to one of Claims 1 to 15.

17. Apparatus (1) according to Claim 16,
wherein the first light source (6) is configured to emit polarized light (16).

18. Apparatus (1) according to Claim 16 or 17,
wherein the speckle device (10) comprises a polarization discriminator (21) and wherein the control unit (13) is configured to detect the change in the speckle pattern (17) for a specific polarization in each case.

19. Apparatus (1) according to one of Claims 16 to 18,
wherein a magnetic field generator (26) for generating a magnetic field at the location where the body (3) is irradiated by light (16) is additionally comprised.

20. Apparatus (1) according to one of Claims 16 to 19,
wherein a tunable light source (6), in particular a semiconductor laser, is comprised as first light source (6).

21. Apparatus (1) according to one of Claims 16 to 20,
wherein the first light source (6) is embodied to generate coherent light (16) and wherein the speckle device (10) is configured to generate and observe the speckle pattern (17) in the light (16) of the first light source (6) scattered at the body (3).

22. Apparatus (1) according to one of Claims 16 to 21,
wherein a second light source (7) for generating coherent light (18), in particular in the visible spectral range, is comprised and wherein the speckle device (10) is configured to generate and observe the speckle pattern (17) in the light (18) of the second light source (7) scattered at the body (3).

## Revendications

1. Procédé de détermination non-invasive de la concentration d'un analyte organique dans un corps biologique (3), dans lequel le corps (3) est automatiquement irradié localement avec de la lumière (16) d'une gamme de longueurs d'onde entre 6 µm et 16 µm adaptée à une signature d'absorption, ou empreinte, de l'analyte, dans lequel au moins une partie de la lumière pénètre dans le corps (3) et est absorbée par l'analyte, dans lequel le corps (3) s'échauffe au moins localement suite à l'absorption par l'analyte et une altération en résultant dans un motif de taches (17) est saisie par de la lumière cohérente (16, 18) diffusée sur le corps (3), et dans lequel une valeur de concentration de l'analyte est déduite de l'altération ayant été saisie dans le motif de taches (17).

2. Procédé selon la revendication 1, dans lequel une déformation de la surface (4) du corps (3), un changement des propriétés de diffusion des zones du corps (3) proches de la surface et/ou un changement de l'indice de réfraction des zones du corps (3) proches de la surface se produisent suite à l'absorption par l'analyte, et sont saisis en tant qu'altération dans le motif de taches (17) .

3. Procédé selon la revendication 1 ou 2, dans lequel le corps (3) est irradié avec de la lumière (16) d'une gamme de longueurs d'onde entre 7 µm et 11 µm.

4. Procédé selon l'une des revendications précédentes, dans lequel la longueur d'onde de la lumière projetée (16) varie pendant la mesure et l'altération dans le motif de taches (17) est saisie respectivement pour différentes longueurs d'onde, dans lequel il est de préférence prévu que la lumière (16) soit projetée alternativement avec une longueur d'onde dans un maximum d'absorption de l'analyte et avec une longueur d'onde entre les maxima d'absorption de l'analyte.

5. Procédé selon l'une des revendications précédentes, dans lequel la densité de puissance de la lumière projetée (16) varie pendant la mesure, et dans lequel l'altération dans le motif de taches (17) est saisie respectivement pour différentes densités de puissance.

6. Procédé selon l'une des revendications précédentes, dans lequel la lumière (16) est projetée périodiquement avec une fréquence de modulation et il est en particulier prévu que la saisie de l'altération dans le motif de taches (17) soit synchronisée sur la fréquence de modulation de la lumière (16) projetée périodiquement.

7. Procédé selon l'une des revendications précédentes, dans lequel l'altération dans le motif de taches (17) est saisie en synchronisation avec une fréquence cardiaque du corps biologique (3).

8. Procédé selon l'une des revendications précédentes, dans lequel une séquence d'images de motifs de taches (17) est capturée et l'altération dans le motif de taches (17) est saisie par une comparaison d'images issues de la séquence d'images capturée, dans lequel l'altération dans le motif de taches (17) est déterminée de préférence comme une valeur de contraste dans une image de différence, et dans lequel il est en particulier prévu que les images de la séquence d'images sont capturées avec une fréquence de rafraîchissement et la fréquence de rafraîchissement est synchronisée à la fréquence de modulation de la lumière (16) projetée périodiquement et/ou à la fréquence cardiaque du corps biologique (3), et dans lequel l'altération dans le motif de taches (17) est saisie à partir de la séquence d'images capturée de préférence comme une fonction du temps.

9. Procédé selon l'une des revendications précédentes, dans lequel le corps (3) est irradié avec de la lumière polarisée (16).

10. Procédé selon l'une des revendications précédentes, dans lequel le motif de taches (17) est observé en fonction de la polarisation de la lumière (16, 18) diffusée sur le corps (3), et dans lequel l'altération dans le motif de taches (17) est saisie respectivement pour une polarisation déterminée.

11. Procédé selon l'une des revendications précédentes, dans lequel un champ magnétique est en outre produit à l'endroit de l'irradiation du corps (3) avec de la lumière (16).

12. Procédé selon l'une des revendications précédentes, dans lequel une source lumineuse accordable (6), en particulier un laser semi-conducteur, est utilisée pour produire la lumière (16) adaptée à la signature d'absorption de l'analyte.

13. Procédé selon l'une des revendications précédentes, dans lequel une première source lumineuse cohérente (6) est utilisée pour produire la lumière (16) adaptée à la signature d'absorption de l'analyte, et dans lequel le motif de taches (17) est observé dans la lumière (16) de la première source lumineuse (6) réfléchie par le corps (3) .

14. Procédé selon l'une des revendications précédentes, dans lequel le corps (3) est irradié avec de la lumière (18) d'une deuxième source lumineuse cohérente (7), en particulier dans le spectre visible, et dans lequel le motif de taches (17) est observé dans la lumière (18) de la deuxième source lumineuse (7) réfléchie par le corps (3) .

15. Procédé selon l'une des revendications précédentes, dans lequel l'analyte considéré est le glucose.

16. Appareil (1) de détermination non-invasive de la concentration d'un analyte organique dans un corps biologique (3), comprenant au moins une première source lumineuse (6) pour irradier le corps (3) avec de la lumière (16) d'une gamme de longueurs d'onde entre 6 µm et 16 µm adaptée à une signature d'absorption, ou empreinte, de l'analyte, un dispositif de taches (10) pour produire et observer un motif de taches (17) de lumière cohérente (16, 18) diffusée sur le corps (3), et une unité de commande (13), lequel est disposé pour saisir une altération dans le motif de taches (17) observé et déduire une valeur de concentration de l'analyte à partir de l'altération ayant été saisie, dans lequel l'unité de commande (13) est en particulier disposée et réalisée pour la mise en œuvre d'un procédé selon l'une des revendications 1 à 15.

17. Appareil (1) selon la revendication 16, dans lequel la première source lumineuse (6) est disposée pour l'émission de lumière polarisée (16).

18. Appareil (1) selon la revendication 16 ou 17, dans lequel le dispositif de taches (10) comprend un discriminateur de polarisation (21), et dans lequel l'unité de commande (13) est disposée pour saisir l'altération dans le motif de taches (17) respectivement pour une polarisation déterminée.

19. Appareil (1) selon l'une des revendications 16 à 18, dans lequel est en outre compris un générateur de champ magnétique (26) pour la production d'un champ magnétique à l'endroit de l'irradiation du corps (3) avec de la lumière (16).

20. Appareil (1) selon l'une des revendications 16 à 19, dans lequel est comprise en tant que première source lumineuse (6) une source lumineuse accordable (6), en particulier un laser semi-conducteur.

21. Appareil (1) selon l'une des revendications 16 à 20, dans lequel la première source lumineuse (6) est réalisée pour produire de la lumière cohérente (16), et dans lequel le dispositif de taches (10) est disposé pour produire et observer le motif de taches (17) dans la lumière (16) de la première source lumineuse (6) diffusée par le corps (3).

22. Appareil (1) selon l'une des revendications 16 à 21, dans lequel est comprise une deuxième source lumineuse (7) pour produire de la lumière cohérente (18), en particulier dans le spectre visible, et dans lequel le dispositif de taches (10) est disposé pour produire et observer le motif de taches (17) dans la lumière (18) de la deuxième source lumineuse (7) diffusée par le corps (3) .
